(19)

Europäisches
Patentamt
European
Patent Office
Office européen
des brevets

(11) **EP 4 227 948 A1**

(12) **EUROPEAN PATENT APPLICATION**

(43) Date of publication:
**16.08.2023 Bulletin 2023/33**

(21) Application number: **22155787.9**

(22) Date of filing: **09.02.2022**

(51) International Patent Classification (IPC):
***G16B 25/10*** (2019.01)       ***G16B 40/20*** (2019.01)

(52) Cooperative Patent Classification (CPC):
**G16B 25/10; G16B 40/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Université de Genève
1211 Genève 4 (CH)**

(72) Inventors:
 • **FABRE, Pierre Jean-Louis Raymond
   1148 L'Isle (CH)**
 • **PRADOS,  Julien Bruno
   74380 Lucinges (FR)**
 • **LO GIUDICE, Quentin Wilhelm Raoul Ange Marie
   74100 Ambilly (FR)**

(74) Representative: **ETL IP
Patent- und Rechtsanwaltsgesellschaft mbH
Clayallee 343
14169 Berlin (DE)**

(54) **MACHINE-LEARNING BASED PREDICTION OF THE SURVIVAL POTENTIAL OF CELLS**

(57)      Techniques for predicting the survival potential of a cell at single-cell resolution are disclosed. Embodiments include a computer-implemented method, a machine-learning model data structure, a computer-implemented method of training a machine-learning model, as well as corresponding computer programs and data processing systems. One embodiment concerns a computer-implemented method (110) of predicting the survival potential of a cell at single-cell resolution. The method comprises receiving an input dataset (112), wherein the input dataset (112) comprises omics data associated with at least one cell of a biological sample. The method further comprises inferring (116) a numerical score which represents a predicted survival potential of the at least one cell using a trained machine-learning model. The method further comprises generating an output dataset (122) comprising the numerical score.

Fig. 3

EP 4 227 948 A1

**Description**

**TECHNICAL FIELD**

The subject-matter disclosed herein generally relates to the field of data-driven medicine, more specifically to the computer-aided analysis of the health level of single-cells at single cell level, and even more specifically to techniques for predicting a survival potential of a cell.

**BACKGROUND**

Neurogenerative diseases affect millions of people worldwide. A neurodegenerative disease is typically caused by the progressive loss of structure or function of neurons, in the process known as neurodegeneration, which may ultimately lead to cell death. Examples of neurogenerative diseases include Alzheimer's disease, Parkinson's disease, frontotemporal dementia (FTD) and amyotrophic lateral sclerosis (ALS). FTD and ALS are progressive neurological disorders that involve cognitive deficits, language abnormalities and muscle weakness. They mostly occur between the age of 40 to 70 and typically lead to death within three to eight years after the first symptom. The estimated number of 2020 ALS cases across 22 countries is 121,028 prevalent and 41,128 incident cases (Brown, Carolyn A., Cathy Lally, Varant Kupelian, and W. Dana Flanders. 2021. "Estimated Prevalence and Incidence of Amyotrophic Lateral Sclerosis and SOD1 and C9orf72 Genetic Variants." Neuroepidemiology 55 (5): 342-53. https://doi.org/10.1159/000516752.), and no treatments are available to stop the degenerative process.

Some studies even claim that as much as one third of the population in Europe, which amounts to roughly 180 million persons, was affected by brain disorders in 2010 ( Wittchen, H.U., F. Jacobi, J. Rehm, A. Gustavsson, M. Svensson, B. Jönsson, J. Olesen, et al. 2011. "The Size and Burden of Mental Disorders and Other Disorders of the Brain in Europe 2010." European Neuropsychopharmacology 21 (9): 655-79. https://doi.org/10.1016/j.euroneuro.2011.07.018.). In the report "2021 Alzheimer's Disease Facts and Figures" of the Alzheimer's Association (Alzheimers Dement 2021; 17(3)), the number of Americans with Alzheimer's disease is estimated to be as many as 6.2 million while the Parkinson's Foundation estimates that 1.2 million people in the United States could be living with Parkinson's disease by 2030 (see https://www.parkinson.org/Understanding-Parkinsons/Statistics).

The current solutions for diagnosing neurogenerative diseases are primarily based on the identification of cell death when the cells are already dying. Moreover, the known methods can typically classify the cells as only "live" or "dead". However, this approach is limited for determining whether a given treatment has an effect on cells, one reason being that it takes a long time with conventional methods to see how the cell reacts.

Unrelated to neurogenerative diseases, WO 2021/127610 A1 is concerned with techniques for prognosing and diagnosing cancer and proposes a method of determining a cancer progression risk score of a subject. The method may include detecting expression levels of genes of a progression gene signature in a sample obtained from a tumor, tissue, or body fluid, and calculating the cancer progression risk score based thereon.

WO 2021/138548 A1 discloses methods for longevity-related applications. It suggests microscopy imaging techniques for predicting a cell's state, function or age based on features such as morphology or expression of certain biomarkers as detected by specific binding reagents. Methods for identifying a drug capable of changing a cell's state, function or predicted age are also provided and considered useful for drug discovery.

US 2014/0057258 A1 discloses a method to generate classifiers to diagnose osteoarthritis in patient samples. Further technological background in the field of degenerative diseases is included in, for example, F. Arai et al.: "Machine Learning of Hematopoietic Stem Cell Divisions from Paired Daughter Cell Expression Profiles Reveals Effects of Aging on Self-Renewal" (Cell Syst. 2020 Dec 16;11(6):640-652.e5. doi: 10.1016/j.cels.2020.11.004. Epub 2020 Dec 8. PMID: 33296684). KR 102144719 B1 titled "Parkinson's disease diagnosis apparatus based on AI (artificial intelligence) using multiple prediction result by multiple learning models, and method", CN 110222745 B titled "Similarity learning based and enhanced cell type identification method", WO 2021/163706 A1 titled "Panomic genomic prevalence score" and Szalai, Bence, Vigneshwari Subramanian, Christian H Holland, Robert Alföldi, László G Puskas, and Julio Saez-Rodriguez. 2019. "Signatures of Cell Death and Proliferation in Perturbation Transcriptomics Data-from Confounding Factor to Effective Prediction." Nucleic Acids Research 47 (19): 10010-26. https://doi.org/10.1093/nar/gkz805.

In view of the above, it is a problem underlying the invention to provide techniques which allow a more high-resolution assessment of the health of a cell, in particular its survival potential.

**SUMMARY**

Techniques for predicting the survival potential of a cell at single-cell resolution are disclosed herein. The cell may be part of a biological sample, e.g., a cell sample comprising mammalian cells, in particular human cells which may have been obtained in a biopsy. Embodiments of the disclosed subject-matter include a computer-implemented method of predicting the survival potential of a cell, a computer-implemented method of training a machine-learning model, a corresponding machine-learning model data structure, as well as corresponding computer programs and data processing systems.

Certain embodiments are based on the observation that the survival potential of the cells composing a sample (e.g. a tissue) may not be uniform, but depend for example on the cell type. Certain embodiments can track the survival potential at single cell level and allow the identification of clusters of "unhealthy" cells, making it highly valuable to investigate the effects of drugs on cell subpopulations.

Although embodiments of the invention are disclosed herein in the context of predicting the survival potential, the concepts disclosed herein are more generally applicable to predicting a health level of a cell, preferably at single-cell resolution, a health level being any parameter associated with or relevant for the health of a cell. Examples of cell's health level-related information besides the survival potential include, without limitation, capacity of proliferation, differentiation, synaptic activity, ion transport activity, regeneration speed, age, and / or metabolism.

Certain embodiments of the invention may be based on the usage of machine-learning model and/or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used that is inferred from an analysis of historical and/or training data. For example, the content of data relating to cell samples or other biological samples may be analyzed using a machine-learning model or using a machine-learning algorithm. In order for the machine-learning model to analyze the content of the data, the machine-learning model may be trained using training data as input and training content information as output. By training the machine-learning model with a large number of training data and associated training content information (e.g. labels or annotations), the machine-learning model "learns" a transformation between the input data and the output, which can be used to provide an output based on non-training data provided to the machine-learning model. The provided data may be preprocessed to obtain a feature vector, which is used as input to the machine-learning model. An innovative aspect in applying machine learning techniques in embodiments of the invention involves framing the prediction problem appropriate to the given task and building the appropriate prediction model for the given task.

One embodiment of a computer-implemented method serves for predicting the survival potential of a cell at single-cell resolution. The method may comprise receiving an input dataset. The input dataset may comprise omics data associated with at least one cell, multiple cells, or even all cells of a biological sample. Omics data may comprise any information associated with a cell or any parameter of a cell which is usable for inferring or determining a survival potential of the respective cell. Generally, the branches of science known informally as -omics are various disciplines in biology whose names end in the suffix - omics, such as genomics, epigenomics, microbiomics, lipidomics, proteomics, glycomics, foodomics, metabolomics, metagenomics and transcriptomics. Accordingly, the -omics data may in one aspect comprise data associated with at least one of a transcriptomic profile, a proteomic profile and an epigenomic profile of the cell.

As one specific example, the input dataset may comprise a transcriptomic profile which comprises a gene expression profile at single-cell resolution. In addition, or alternatively, the transcriptomic profile may comprise single-cell mRNA sequencing data.

The input dataset profiles are preferably in h5 or FASTQ format, and may also comprise metadata (preferably text format). The text may be converted into a format suitable for further processing, such as into a comma-separated values (CSV) format.

The method may comprise inferring a numerical score, also referred to herein as "survival outcome score" or "SOS", which represents a predicted survival potential value of the at least one cell using a trained machine-learning model, in particular using any machine-learning model disclosed herein. A numerical score may be inferred for each one of several selected cells or one numerical score may be inferred per cell of the cell sample. It is also conceivable to infer a combined numerical score for a group of cells, or only for a selected subset of cells as representatives of the cell sample. The method may generate an output dataset comprising the numerical score or scores. The numerical score may be a number in the form of an integer or, more preferably, a floating-point number. The numerical score may correspond to or fall within one of several classes on a scale, non-limiting examples of which include "dead", "ongoing cell death", "weak", "robust" and/or "regenerative".

Accordingly, the method departs from the known approaches in which a cell can be classified as either dead or alive, and provides a more proactive, forward-looking approach where the survival potential is predicted and effectively forecasted.

The method may further comprise generating a unified representation of data of the input dataset, in particular a gene expression matrix based on at least part of the input dataset. The gene expression matrix may encode a normalized expression of a given gene in a given cell, preferably for all cells under consideration. In one aspect, if the input dataset comprises a transcriptomic profile, the method may convert the transcriptomic profile into a uniformized matrix representation which encodes a (normalized) expression of a given gene in a given cell. The following is an illustrative non-limiting example of a gene expression matrix:

|  | Cell 1 | Cell 2 | ... | Cell n |
|---|---|---|---|---|
| Gene 1 | 3.7 | 2.0 | ... | 13.8 |
| Gene 2 | 2.2 | 3.2 | ... | 1.1 |

(continued)

|  | Cell 1 | Cell 2 | ... | Cell n |
|---|---|---|---|---|
| Gene 3 | 1.6 | 14.4 | ... | 18.6 |
| ... | ... | ... | ... | ... |
| Gene m | 25.5 | 0.0 | ... | 0.0 |

The gene expression matrix is preferably encoded in a matrix representation. The matrix may be provided in a machine-readable format such as the Hierarchical Data Format (HDF), e.g. HDF4 or HDF5, comma-separated values (CSV), tab-separated values (TSV), Matrix Market (MM), a 3-columns sparse matrix representation, or the like.

The generated gene expression matrix may serve as the input for inferring the numerical score (the "survival outcome score") described above. Using the gene expression matrix as input for the machine-learning model has the advantage that data can be structured into biologically relevant units of information, and such a matrix can be generated in many contexts which provides applicability of the model in a broad range of situations.

Generating the gene expression matrix may comprise at least one of:

- removing a cell if an associated number of expressed genes of the cell is below a predefined threshold

- removing a cell if a unique molecular identifier (UMI) count of the cell is below a predefined threshold

- removing a cell if an associated number of expressed genes is outside a global normal distribution of the number of expressed genes in the input dataset

- removing a cell if an associated magnitude of expressed genes is outside a global normal distribution of the magnitude of expressed genes in the input dataset

- removing a gene if a number of cells in which the gene is expressed is below a predefined threshold

- removing a gene if the expression magnitude of a gene is steady across a number of cells

- homogenizing, in particular by down-sampling, an amount of UMIs per cell to be within a predefined range

- reorganizing matrix rows and/or matrix columns according to gene names

- identifying missing genes and, optionally, performing a remedying action (e.g. filling the missing entry with a specific value, such as an average value or a value computed by an imputation method)

- adjusting expression values so they are expressed into an identical unit (e.g. log-transformation and/or scaling by a pre-defined (or computed) normalization factor and/or variable standardization, gene length normalization and/or expression centering).

The input dataset may be derived from different sources. One example includes a nucleic acid sequence count, preferably in FASTA/FASTQ format, generated by a sequencing instrument. Generating the gene expression matrix may comprise processing a cell barcode in the nucleic acid sequence to identify the cell of origin, and comparing the nucleic acid sequence to a reference genome to identify the gene or genes of origin.

Another example involves gene expression values which are already organized as a matrix, which may be obtained from multiple sources including: processed sequencing data, gene expression values.

The machine-learning model may comprise a deep-learning model and an ordinal regression model. Inferring the numerical score may comprise processing the input dataset by the deep-learning model and the ordinal regression model, preferably in parallel, and generating the numerical score based on a combination of outputs of the deep-learning model and the ordinal regression model. The outputs may not be directly combined, but the values may be first standardized independently on the datasets. In certain embodiments, two pieces of information are pertinent when combining: the average score and the difference between scores. The combination of these two types of models is particularly beneficial because an advantage of the ordinal regression model is that the model is easy to interpret. Compared to the deep-learning model, the ordinal-regression may provide better insight on the genes that are used to compute the SOS score. The ordinal regression model was in come embodiments used to identify a reduced list of genes used to compute the survival score, onto which biological hypothesis underlying embodiments of the invention are built. A drawback of the ordinal regression model may be that it is more sensitive to noise in the input data. In contrast, the deep-learning model can be designed to be more robust to noise in the input dataset because of a number of factors: (1) The model can use all input genes to compute the SOS score, and can then aggregate this information to correct a noisy input; (2) the deep-learning model can be trained with noisy input data to push the network to produce robust scores; (3) the deep-learning model may be designed to be robust to variations in the normalization of the input: by introducing a feed-back loop the network normalizes differently distinct views of the input data. A drawback of the deep-learning model may be its interpretability: as the expression of virtually all genes may be used by the model, it may be more difficult to extract a reduced list of genes to extrapolate a biological hypothesis like for the ordinal-regression model.

The method may further comprise generating a confidence index which indicates an expected variability between the outputs of the deep-learning model and the ordinal regression model. The output dataset may comprise the confidence index. This way, it is possible to evaluate the quality of the scores produced by the method.

The method may further comprise displaying the output dataset on an electronic display device. The displaying may comprise displaying the numerical values directly and/or displaying a graphical representation of the numerical score. The predicted survival potential may be indicated by a color associated with the numerical score and/or by its position on a scale. Accordingly, this allows for an easy to perceive and intuitively to understand use of the scores.

The method may further comprise repeating at least a subset of the steps disclosed above with at least one additional input dataset associated with the same subject to generate at least one additional output dataset. The output dataset and the at least one additional output dataset may be compared. This allows to derive meaningful insights into the development of the subject over time, e.g. for monitoring the course of a disease or the effectiveness of a drug.

In one embodiment, a machine-learning model data structure is provided. Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g., based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g., of the sum of its inputs). The inputs of a node may be used in the function based on a "weight" of the edge or of the node that provides the input.

The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e., support vector networks) are supervised learning models with associated learning algorithms that may be used to analyze data (e.g., in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph.

Alternatively, the machine-learning model may be based on a genetic algorithm, which is a search algorithm and heuristic technique that mimics the process of natural selection.

The machine-learning model may be configured for predicting the survival potential of a cell at single-cell resolution, particular in any of the methods disclosed herein. The machine-learning model may comprise an input module configured for receiving an input dataset and an output module configured to produce an output dataset. The input dataset may comprise omics data associated with at least one cell of a cell sample. The output dataset may comprise a numerical score which represents a predicted survival potential of the at least one cell.

The machine-learning model may comprise a deep-learning model and an ordinal regression model. The machine-learning model may comprise a combiner module for combining outputs of the deep-learning model and the ordinal regression model to generate the numerical score or scores.

The deep-learning model may comprise an input layer comprising a plurality of nodes. Each node may be associated with a particular gene of a cell. The deep-learning model may comprise a first hidden layer comprising a plurality of nodes i, each node i being associated with a linear weight $w_i$ with a normalization constraint of $1 : \|w_i\| = 1$, and wherein x denotes the input of the first hidden layer (206) and the first hidden layer (206) is configured for dividing the outputs $<w_i.x>$ by $d = <|w_i|.|x|>$. The deep-learning model may comprise an output layer comprising a node associated with a numerical score which represents the predicted survival potential of the cell. This allows the normalization to be more efficient. The normalization considerably improves the adaptation of the model to new datasets.

The ordinal regression model may comprise a loss function of the following form:

$$ordinal_{loss(f,y)} = \max_{\forall(i,j)|y_i < y_j} (0,1 + f_i - f_j)$$

The output dataset produced by one of the machine-learning models disclosed herein may be used for a variety of use cases. Non-limiting examples include:

- predicting an effectiveness of a candidate agent for a degenerative disease

- assessing the level of cytotoxicity in a population of cells

- indicating the severity of a degenerative disease to improve prognosis

- identifying which particular cell types are responding to a given treatment

- assessing the health status of a single cell

In one embodiment, a computer-implemented method of training a machine-learning model is provided. The machine-learning model may be configured for predicting the survival potential of a cell sample at single-cell resolution, in particular in accordance with any of the characteristics disclosed herein.

The method may comprise receiving a training dataset. The training dataset may comprise omics data associated with a plurality of cells and information on the health status of the plurality of cells. The method may comprise training the machine-learning model using the training dataset or data associated with the training dataset.

Machine-learning models may be trained using training input data. The above example uses a training method called "supervised learning". In supervised learning, the machine-learning model is trained using a plurality of training samples, wherein each sample may comprise a plurality of input data values, and a plurality of desired output values, i.e., each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g., a classification algorithm, a regression algorithm or a similarity learning algorithm). Classification algorithms may be used when the outputs are restricted to a limited set of values (categorical variables), i.e., the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are.

Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (predefined) similarity criteria, while being dissimilar to input values that are included in other clusters.

Reinforcement learning is a third group of machine-learning algorithms that may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e., outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g., a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

The training dataset may be obtained in the context of a biological experiment of one of the following designs comprising:

- a perturbation experiment where beneficial and/or aggressive treatments are applied to cells, such as mechanical aggression and their transcriptomic response is followed over time together with their health status, such as e.g. cell death and/or cell proliferation

- a regeneration experiment where transcriptomic profiles of cells from regenerative tissues are followed over time so the regenerative ability of a cell can be estimated from its transcriptomic profile

- a degeneration experiment where the transcriptomic profiles of cells into degenerative diseases is compared or followed over time

- an aging experiment where transcriptomic profiles of cells into organisms of increasing age is followed or compared between specimens so as to link the impact of age on the cell transcriptome

- a developmental experiment where transcriptomic profiles of cells are followed during development of an organism

The training method may further comprise generating a gene expression matrix based on at least part of the training dataset, wherein the gene expression matrix encodes a normalized expression of a given gene in a given cell. The gene expression matrix may be identical, or at least similar, to the gene expression matrix used during the inference method described further above. The generated gene expression matrix may serve as the input for the training method.

The training of the machine-learning model may comprise subjecting a deep-learning model of the machine-learning model to a first training phase. The first training phase may comprise a first plurality of epochs, preferably at least 30 epochs. The training may comprise determining a pruning mask for a first hidden layer of the deep-learning model. The pruning mask may be configured for selecting a first set of nodes with the highest weights and a second set of nodes with the lowest weights. The training may comprise subjecting the deep-learning model to a second training phase with the pruning mask applied. The second training phase may comprise a second plurality of epochs, preferably at least 30 epochs. The pruning serves to simplify the model against overfitting and to make the network more robust to global normalization changes.

Preferably, a dropout probability of the first hidden layer involved in the first and/or second training step may be selected from the range of 30% to 70%, more preferably from the range of 40% to 60%, and most probably is a dropout probability of essentially 50%. With a dropout probability of 50%, half of the values are hidden during the training process, which is in certain embodiments considered to be a good estimate of what one may expect in a new dataset. Setting the dropout probability lower may not make the output robust enough to noise, setting it higher may require lot of epochs in the training process.

Some embodiments of the invention provide a data carrier having electronically readable control signals, which are capable of cooperating with a programmable computer system, such that one of the methods described herein is performed.

Generally, embodiments of the invention can be implemented as a computer program (product) with a program code, the program code being operative for performing one of the methods when the computer program product runs on a computer. The program code may, for example, be stored on a machine-readable carrier. Other embodiments comprise the computer program for performing one of the methods described herein, stored on a machine-readable carrier. In other words, an embodiment of the present invention is, therefore, a computer program having a program code for performing one of the methods described herein, when the computer program runs on a computer.

A further embodiment of the invention provides a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention provides a data stream or a sequence of signals representing the computer program for performing one of the methods described herein. The data stream or the sequence of signals may, for example, be configured to be transferred via a data communication connection, for example, via the internet.

A further embodiment of the invention provides a processing means, for example, a computer or a programmable logic device, configured to, or adapted to, perform one of the methods described herein.

A further embodiment of the invention provides a computer having installed thereon the computer program for performing one of the methods described herein.

A further embodiment of the invention provides an apparatus or a system configured to transfer (e.g., electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

**BRIEF DESCRIPTION OF THE DRAWINGS**

The disclosure may be better understood by reference to the following drawings:

Fig. 1:    A flowchart illustrating a machine-learning phase and an inference phase in accordance with embodiments of the invention

Fig. 2:    A conceptual representation of a deep learning model usable in embodiments of the invention

Fig. 3:    A graphical representation of survival outcome scores in accordance with embodiments of the invention as compared to the prior art

Fig. 4:    Validation results to exemplify the robustness of survival outcome scores calculated for a sample of cells in accordance with embodiments of the invention

Fig. 5:    Validation results to exemplify the robustness of survival outcome scores obtained by a 200-genes classifier in accordance with embodiments of the invention

**DESCRIPTION OF PREFERRED EMBODIMENTS**

In the following, various techniques for predicting the survival potential of a cell at single-cell resolution will be described. The disclosed techniques may be used for identifying a candidate agent that may be effective to treat or ameliorate an effect of a (neuro)degenerative disease in a subject, or for a specific treatment.

In one embodiment, the overall process may include a database creation phase, a model building phase and a health score inference phase. The database creation phase may include extracting a large dataset from single-cell RNA sequencing data. The database creation phase may also include filtrating and/or normalizing the data. The model building phase may include building a classifier based on a machine-learning strategy. The model building phase may also include validating the classifier with cells which respond positive or negative to the treatment under consideration. The health score inference phase may include algorithmically identifying cells which respond to the treatment to ultimately accelerate the discovery of a suitable drug.

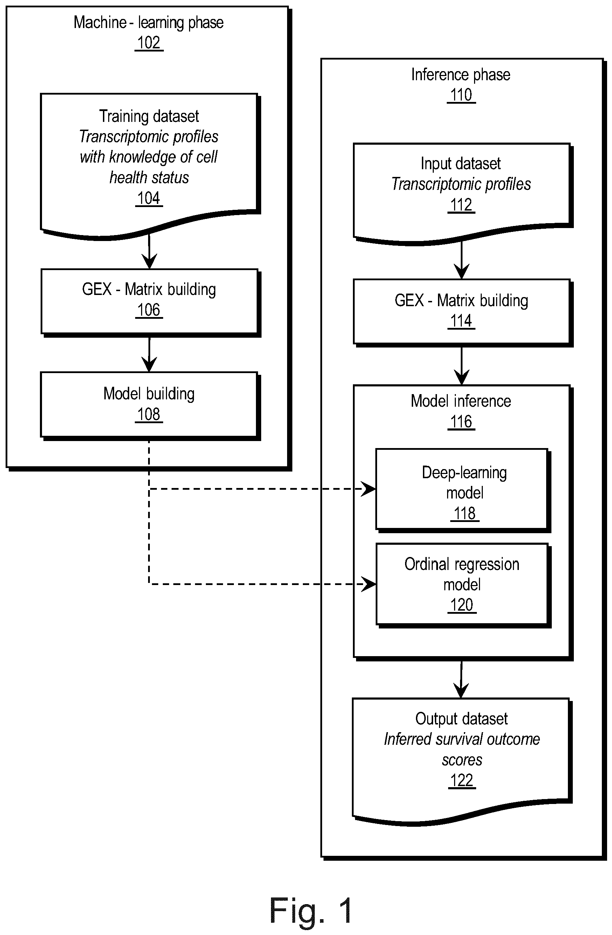

Fig. 1 illustrates an overall workflow according to an embodiment including a machine-learning phase 102 and an inference phase 110. The overall purpose of the machine-learning phase 102 is to build a suitable model from training data, and the purpose of the inference phase 110 is to use the model on real input data to predict the survival potential. It shall be understood that the machine-learning phase 102 and the inference phase 110 may be performed by different entities at different points in time, independent of each other.

In the following, the embodiment of the machine-learning phase 102 shown in Fig. 1 will first be described. The machine-learning phase 102 operates on a training dataset 104 as input. In the illustrated example, the training dataset 104 comprises transcriptomic profiles and information about the cell health status. The data may comprise raw datasets as FASTQ, for example, which have been produced using single-cell genomics techniques such as Drop-seq, Nuc-seq or 10X genomics scRNA-seq. The data may be converted to a computer-readable format, such as comma-separated values (CSV).

The cell names may be barcoded. In single-cell transcriptomics, barcoding of the cell typically involves (1) isolating cells into droplets, wells of a plate, or using a microfluidic system, (2) lysing and extracting RNA from each cell, and (3) adding a unique nucleic-acid sequence (the barcode) into each isolate to the transcripts (usually by reverse-transcription). The barcode will typically be sequenced together with the transcripts during sequencing.

In the example, the data has been produced using different genomics methods, and thus a customization step is performed for every set of data to ensure proper normalization. To this end, as illustrated in Fig. 1, the machine-learning phase 102 includes a matrix building step 106. In the example, the matrix (also referred to herein as gene expression matrix or GEX matrix) is generated by applying defined filters. Cells that are expressing a too low number of genes and UMIs (<500 = cellular debris) are removed, and cells that are outside the global normal distribution of the number of expressed genes (cell aggregation or doublets) are removed. Furthermore, genes that are expressed in a low fraction of the cells are removed as they cannot represent any meaningful variations or biological features. The number of UMIs per cell within the acceptable thresholds is homogenized by down sampling to generate gene expression profiles that can be comparable. The resulting gene expression matrix is composed of the gene expression of each cell present in the dataset.

The generated gene expression matrix is then used in the model building step 108 illustrated in Fig. 1. In the described embodiment, the machine-learning model is a combination of two different machine-learning models, namely a deep-learning model 118 and an ordinal regression model 120. The ordinal regression model 120 of the illustrated embodiment serves for extracting a list of marker genes, and may be sensitive to input data changes. The deep-learning model 118 of the illustrated embodiment is designed to be highly robust to input changes and thus allows more flexibility to adapt for multimodal datasets, such as single-cell RNA-seq that can be integrated in the analysis as an extra set of parameters.

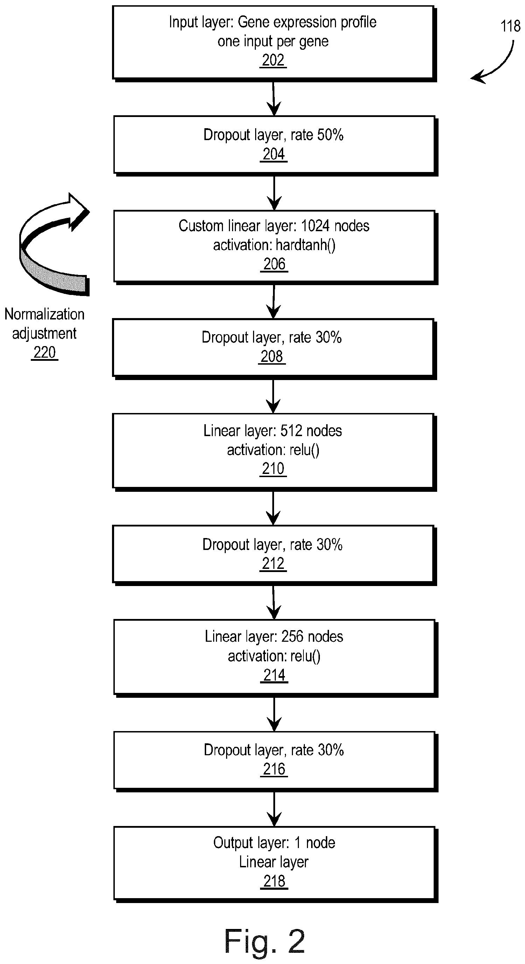

Fig. 2 is a more detailed illustration of the structure of the deep-learning model 118 according to an embodiment. The model 118 comprises an input layer 202, a first custom linear layer 206, a second linear layer 210, a third linear layer 214, an output layer 218, and four dropout layers 204, 208, 212, 216.

The input layer 202 comprises one node per gene. The inputs are expected to be Reads Per Million Reads (RPM) normalized gene expression profiles on a logarithmic scale.

The output layer 218 comprises one node for the survival outcome score.

To make the model 118 robust to missing values, it is simulated with a high dropout rate all along the network and onto the input layer. Accordingly, the model 118 in Fig. 1 comprises a first dropout layer 204 after the input layer 202 with a dropout rate of 50%, and three more dropout layers 208, 212, 216, each with a dropout rate of 30%.

The first linear layer 206 of the model 118 is a custom linear layer which is designed to handle different input normalizations. Let x be the input of layer 206, and $w_i$ the weights for the $i^{th}$ node of layer 206. Layer 206 is designed such that:

- The linear weights of all nodes have a normalization constraint of 1 : $\|w_i\| = 1$

- The outputs $<w_i.x>$ of the linear layer 206 are divided by $\mathbf{d} = <|w_i|.|x|>$ (i.e., the dot product between the absolute values of $w_i$ and the absolute values **of x).**

The rationale behind the customization of layer 206 is that the linear weights $\mathbf{w_i}$ can be seen as a mask on the input values that select a view of the input data. Dividing the linear outputs by $\mathbf{d}$ is a way to dynamically normalize (as represented by the arrow 220 in Fig. 2) the data on a reduced fraction of the input that is considered by node i. This allows the normalization to be more efficient. The inventors have found and validated in experiments that this considerably improves the adaptation of the model to new datasets.

One embodiment of a training process for the model 118 comprises a first training phase in which there are no pruning constraints on layer 206, and the network 118 is trained on at least 30 epochs with an ADAM optimizer. After the first training phase, a pruning mask is computed on layer 206. The pruning serves to analyze the linear weights of layer 206 and to keep, for each node, 100 genes, namely the 50 genes with the highest weights and the 50 genes with the lowest weights. In a second training phase, the network weights are fitted again on at least 30 more epochs with the pruning constraints applied, so the unpruned network weights can adapt to the pruning. The pruning serves to simplify the model 118 against overfitting and to make the network more robust to global normalization changes.

Returning to the model building step 108 illustrated in Fig. 1, The ordinal regression model 120 is, in the illustrated example, based on the "bmrm" R package developed by Julien Prados, which is available at https://github.com/pradosj/bmrm. bmrm is an R package implementing a bundle method for minimization of convex and non-convex risk under L1 or L2 regularization. It implements the algorithm proposed by Teo et al.: "Bundle Methods for Regularized Risk Minimization" (JMLR 2010) as well as the extension proposed by Do and Artieres: "Regularized bundle methods for convex and non-convex risks" (JMLR 201). The package includes various loss functions for machine learning suitable for big data analysis. Applications include structured prediction, linear SVM, multiclass SVM, f-beta optimization, ROC optimization, ordinal regression, quantile regression, epsilon insensitive regression, least mean square, logistic regression, and least absolute deviation regression, all with L1 and L2 regularization. For use in the illustrated embodiment, the preferred loss function used to train the network 118 is the ordinal loss function

$$ordinal_{loss(f,y)} = \max_{\forall (i,j)|y_i < y_j} (0, 1 + f_i - f_j),$$

where f is the vector network predictions for a set of samples, and y is the expected network outcome. The arguments that can be used are as follows:

- x: matrix of training instances (one instance by row)

- y: integer vector of positive values (>=1) representing the training labels for each instance in x

- C: the cost matrix to use, C[i,j] being the cost for predicting label i instead of label j.

- Impl: either the string "loglin" or "quadratic", which defines the implementation to use for the computation of the loss. Value a function taking one argument w and computing the loss value and the gradient at point w

Thus, the modeling step comprises in the illustrated example a regression analysis which is used to predict an ordinal variable. In the present example, the variables are the survivability or the vulnerability of cells following toxic treatments that we obtained from longitudinal studies and from which a genetic signature composed of specific weighted interactions was extracted. The model is applicable to other normalized datasets to infer the survival scores of each cell.

The trained machine-learning model is then used in the inference phase 110. Returning to the example in Fig. 1, the inference takes as input an input dataset 112 with transcriptomic profiles. A gene expression matrix 114 is built in step 114, similar to the gene expression matrix building step 106 performed during the machine-learning phase 102. The generated gene expression matrix is then subjected to the model inference 116, which involves the combined machine-learning model including the deep-learning model 118 and the ordinal regression model 120 built during the machine-learning phase 102.

The output of the model inference step 116 is, in the illustrated example, post-processed as follows: The generated survival outcome scores (one per evaluated cell) are standardized across the cell sample. The respective scores produced by the deep-learning model 118 and the ordinal regression model 120 are merged (in the example by averaging), and the standard deviation is calculated. The variability (e.g., as quantified by the standard deviation) between the two scores is used to define a confidence index. The scores for each cell are associated with their associated cell type. An output dataset 122 (e.g., in the form of a matrix data structure) is generated comprising the above information. Certain embodiments of the invention may also generate an output dataset 122 with only a subset of the above information. In the output dataset 122, the cell names may be barcoded for confidentiality reasons.

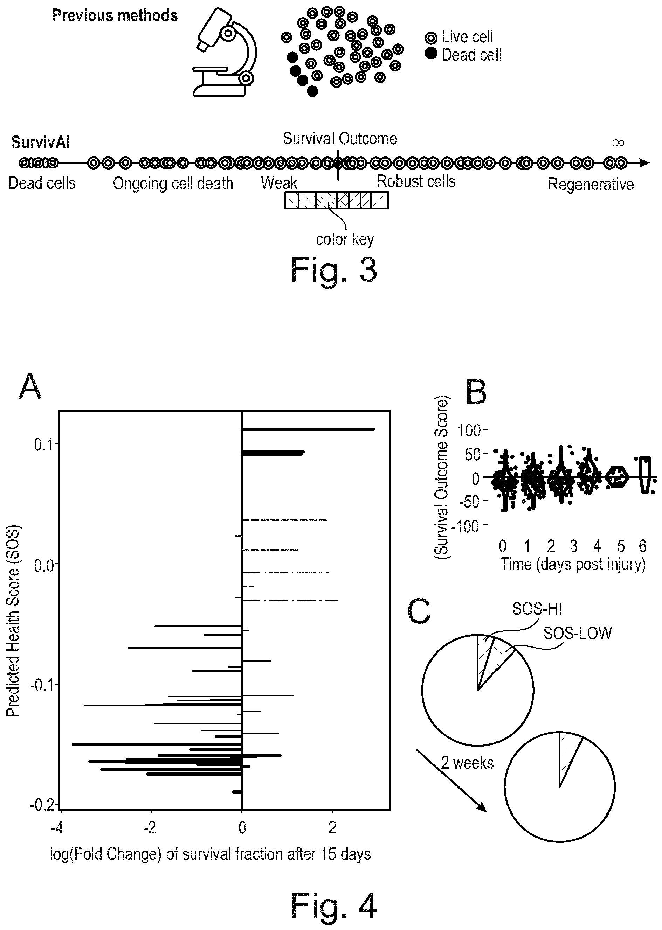

Fig. 3 illustrates in the top section the approach taken predominantly in the prior art, namely to identify a cell as either alive or dead. The bottom section in Fig. 3 illustrates an exemplary visual representation of survival outcome scores according to an embodiment of the invention. As can be seen, the representation comprises a scale of several classes (in the example: "dead cells", "ongoing cell death", "week", "robust cells", "regenerative"). Each dot represents a given cell of the evaluated cell sample. The survival outcome score of a cell may be indicated by its position on the scale, as shown in Fig. 3. The survival score of a cell may also be indicated by the color of the corresponding dot, e.g., purple for "dead cells", orange for "ongoing cell death", brown for "week", dark green for "robust cells" and light green for "regenerative". Also a combination of both position and color is conceivable. While most methods in the prior art assess the viability of the cells by scoring their status as dead (or almost dead) or alive, the single-cell scores computed by embodiments of the invention may reveal a continuum of states that have a predictive value for the capacity of each single cell to survive. Thus, while some cells with poor values are likely to be engaged in a cell death process, other cells may be alive but stated as weak, and yet other cells may be alive and stated as robust, etc.

A proof of principle is provided in Fig. 4. Figs. 4A and 4B are cross-validations on neurons from the retina. Fig. 4A illustrates results of an analysis performed on over forty populations of cells with the scores of the cell at time 0 plotted on the x-axis and a value indicating the overall change of the fraction of cells remaining two weeks after an injury on the same cells plotted on the y-axis. Each line represents a group of hundred single-cells. The figure shows that cells with poor values (towards the lower part of the chart) were depleted over time, while cells with high scores remain and thus represent a larger fraction of the cell population.

Fig. 4B illustrates violin plots showing the progression of a cluster of about one hundred cells with an initial low survival score. Over time, as cells die, the number of cells in the group decreases, with the remining cells being the ones with the higher scores.

Fig. 4C is a validation on cortical neurons (upper motor neurons) from a different dataset. The pie charts represent the proportion of two upper motoneurons populations with high SOS (labelled "SOS-HI") and low SOS (labelled "SOS-LOW") among the general population. These motoneurons are shown three days after injury (upper pie chart) and 15 days after injury (lower pie chart). The remaining population of cells hare a higher degree of molecular identity with the population with higher SOS.

The foregoing is a disclosure of various techniques for determining and predicting the survival potential of cells, e.g., from mammalian cultures or human biopsies. Embodiments provide methods for identifying a candidate agent that may be effective to treat or ameliorate an effect of a degenerative disease in a subject or for a specific treatment. Certain embodiments of the disclosed methods may comprise or use i) a 200-genes classifier ranking and weighting levels of mRNA abundance with ii) a combinatorial series of a top genes signature for specific cell types, and which best explains the classifier performance to identify therapeutical agents.

In one embodiment, the 200-genes classifier / signature is produced by the ordinal regression model 120. The 200-genes classifier may be configured for ranking dynamics of mRNA abundance in cells with different potential of survival in vivo and in vitro. The strength was exemplified with a 32 genes signature (so-called "best-fit signature"; BFS) which best explains the classifier performance. The proof of concept (see Fig. 4) of its generalizability was provided by validating the classifier in three independent single-cell datasets, including public data (Tran et al., 2019) data from a previous publication of the inventors (Lo Giudice et al., 2019) and a large unpublished single-cell dataset (Leleu et al., in preparation).

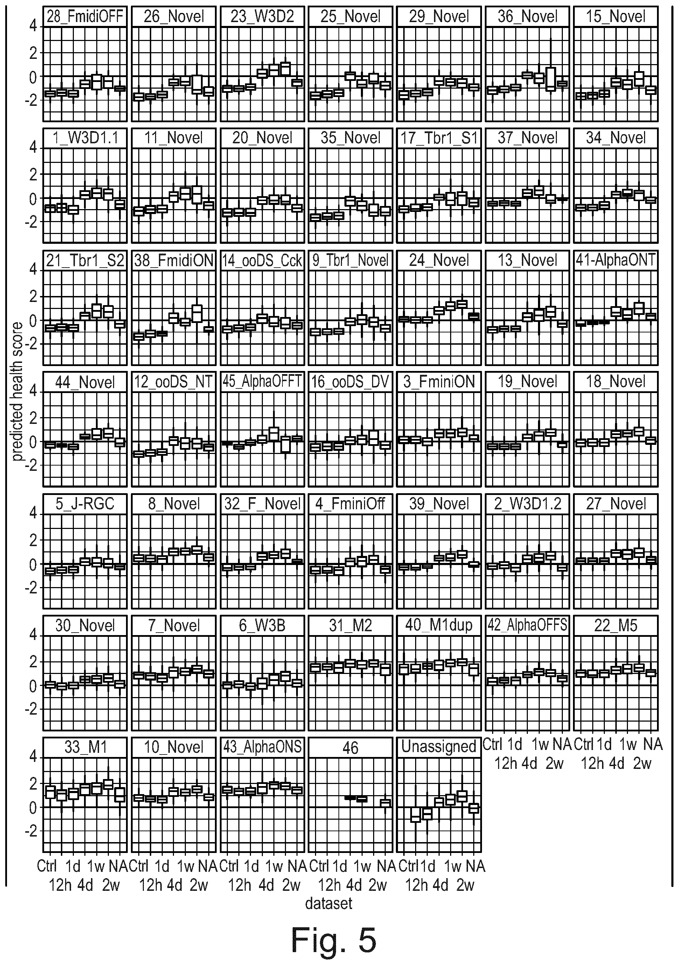

Fig. 5 illustrates the robustness of the survival outcome scores obtained by using the above-mentioned 200-genes classifier on retinal cells subjected to severe injury.

Certain embodiments compute health-scores for cells of any types based on their transcriptomic profile. One application is to compare health scores under different treatment conditions. To compute the scores, one may rely on the outcome of biological experiments which must be carefully designed to capture the expected outcome (cell health). A typical focus is on time course experiments where cells have been treated or aggressed. The cells are followed over time and the survival outcome scores are observed at different points of the experiment. The prediction models disclosed herein are designed with the objective to be able to predict as early as possible the outcomes of cell survivability. The disclosed methods may also be applied to experimental designs related to aging, cell regeneration (e.g., for liver cells), cell duplication (e.g., for cancer-cells) and perturb-seq experiments.

Embodiments of the invention are susceptible to being used with human stem cells and cells derived from patients with cancer or other degenerative diseases. The robustness of embodiments of the method is useful to refine high throughput technologies, including image-based cytometry assay and genetic engineered organoid screening. Embodiments of the disclosed techniques will also be useful for clinical settings by way of its capacity to evaluate strong clinical prognosis of cells. Embodiments of the disclosed techniques benefit from a solid read-out based on its strong sensitivity and predictive value. This allows to rapidly test and identify new drugs and/or biophysical factors which are key to sustain viability in human cells.

Accordingly, the techniques disclosed herein may be used to identify agents that can increase survival ratio for cell-type specific population in animal model and human cultures. The sensitivity of refined parameters is one beneficial characteristic of the disclosed techniques, with each gene having a different impact on the assessment of the survival outcome score. The result of the assessment represents a genetic interrogation of survival and regenerative capacity. With the rapidly growing overall demand for data-driven medicine, the disclosed techniques provide an analytical package for drug and/or treatment screening that is usable by hospitals, pharmaceutical and/or medical companies, as well as academic institutions.

In the following, various non-limiting examples of using the output dataset 122 produced by one of the methods disclosed herein will be described:
Predicting an effectiveness of a candidate agent for a degenerative disease: For example, during therapeutics screening of numerous compounds, while developing a new drug, the compound may be assessed early on, before the classical readouts. While single-cells share many morphological features of viability, they may be at risk based on their vulnerable combinatorial transcriptomes. In a degenerative setting, it may take years for the weakest cells to succumb. A candidate agent that can restore their viability outcome before the cell death can thus be screened since it will increase the survival outcome score (SOS) of these cells. Furthermore, some sub-populations of cells are known to be at risk for particular disease (such as beta-cell failure in diabetes, lower motor neuron loss in spinal muscular atrophy, dopaminergic neurons from the substantia nigra pars compacta in Parkinson disease, retinal ganglion cells in Glaucoma etc). Another example is the rescuing of a particular metabolic pathway which will not change the test cells readouts on a classical compound assay, but that can be detected by embodiments of the present disclosure. Also conceivable is a rescue of a phenotype or readout by adding up the different drugs, "rebuilding" the correct molecular pathway, such as to create an atlas of how to rebuild a pathway.

Assessing the level of cytotoxicity in a population of cells: a given treatment may lower the SOS of some cells, and thus reveal a potential harmful effect of a given drug, thereby allowing the fast identification of dangerous compounds during therapeutics developments.

Indicating the severity of a degenerative disease to improve prognosis: most diseases are not progressing at the same pace, and lower SOS scores are associated with poorer survival rate. By the quantification of the neurodegeneration level, the level and composition of therapeutic drugs may be adapted.

Identifying which particular cell types are responding to a given treatment: Higher SOS is a strong indication that a given population of cells is very robust and thus will have higher survival potential. Cells sharing most of their molecular identity will actually have distinctive SOS and thus can be distinguished on their survival outcome. This may allow research to be done in a more specific way toward a disease or a compound. It may also enable finding cell types not yet known to be reactive for a specific compound, in the sense of a new way of drug screening.

Assessing the health status of a single cell: even in physiological settings one can assess the differential robustness of cells in a large population, which is particularly useful for rare and/or critical cell types in vivo.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some or all of the method steps may be executed by (or using) a hardware apparatus, such as a processor, a microprocessor, a programmable computer or an electronic circuit. Depending on certain implementation requirements, embodiments of the invention can be implemented in hardware or in software. The implementation can be performed using a non-transitory computer-readable storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed.

**Claims**

1. A computer-implemented method (110) of predicting the survival potential of a cell at single-cell resolution, the method comprising:

   receiving an input dataset (112), wherein the input dataset (112) comprises omics data associated with at least one cell of a biological sample;
   inferring (116) a numerical score which represents a predicted survival potential of the at least one cell using a trained machine-learning model, in particular the machine-learning model of any one of claims 6-9; and
   generating an output dataset (122) comprising the numerical score.

2. The method of claim 1, further comprising:

   generating (114) a unified representation of data of the input dataset (112), in particular a gene expression matrix based on at least part of the input dataset (112), wherein the gene expression matrix encodes a normalized expression of a given gene in a given cell;
   wherein generating (114) the gene expression matrix comprises at least one of:

      - removing a cell if an associated number of expressed genes of the cell is below a predefined threshold;
      - removing a cell if a unique molecular identifier, UMI, count of the cell is below a predefined threshold;
      - removing a cell if an associated number of expressed genes is outside a global normal distribution of the number of expressed genes in the input dataset (112);
      - removing a cell if an associated magnitude of expressed genes is outside a global normal distribution of the magnitude of expressed genes in the input dataset (112);
      - removing a gene if a number of cells in which the gene is expressed is below a predefined threshold;
      - removing a gene if the expression magnitude of a gene is steady across a number of cells;
      - homogenizing, in particular by down-sampling, an amount of UMIs per cell to be within a predefined range;
      - reorganizing matrix rows and/or matrix columns according to gene names
      - identifying missing genes and, optionally, performing a remedying action such as filling the missing entry with a specific value, such as an average value or a value computed by an imputation method;
      - adjusting expression values so they are expressed into an identical unit, such as log-transformation and/or scaling by a pre-defined or computed normalization factor and/or variable standardization, gene length normalization and/or expression centering.

3. The method of claim 1 or 2, wherein the input dataset (112) is derived from at least one source comprising:

   a nucleic acid sequence count, preferably in FASTA/FASTQ format, generated by a sequencing instrument, wherein generating (114) the gene expression matrix comprises processing a cell barcode in the nucleic acid sequence to identify the cell of origin, and comparing the nucleic acid sequence to a reference genome to identify the gene or genes of origin;
   gene expression values already organized as a matrix, which may be obtained from multiple sources including: processed sequencing data, gene expression values.

4. The method of any one of the preceding claims, wherein the machine-learning model comprises a deep-learning model (118) and an ordinal regression model (120); and
   wherein inferring (116) the numerical score comprises:

   processing the input dataset (112) by the deep-learning model (118) and the ordinal regression model (120);
   generating the numerical score based on a combination of outputs of the deep-learning model (118) and the ordinal regression model (120);
   generating a confidence index which indicates an expected variability between the outputs of the deep-learning model (118) and the ordinal regression model (120); wherein the output dataset (122) comprises the confidence index.

5. The method of any one of the preceding claims, further comprising displaying the output dataset (122) on an electronic display device;

   wherein the displaying comprises displaying a graphical representation of the numerical score;
   wherein, preferably, the predicted survival potential is indicated by a color associated with the numerical score and/or by its position on a scale.

6. A machine-learning model data structure configured for predicting the survival potential of a cell at single-cell resolution, wherein the machine-learning model comprises:

an input module configured for receiving an input dataset (112), wherein the input dataset (112) comprises omics data associated with at least one cell of a biological sample; and
an output module configured to produce an output dataset (122), wherein the output dataset (104) comprises a numerical score which represents a predicted survival potential of the at least one cell.

7. The machine-learning model of claim 6, further comprising:

a deep-learning model (118);
an ordinal regression model (120); and
a combiner module for combining outputs of the deep-learning model (118) and the ordinal regression model (120) to generate the numerical score.

8. The machine-learning model of claim 7, wherein the deep-learning model (118) comprises:

an input layer (202) comprising a plurality of nodes, each node associated with a particular gene of a cell;
a first hidden layer (206) comprising a plurality of nodes i, each node i being associated with a linear weight $w_i$ with a normalization constraint of $1$:$\|w_i\|=1$, and wherein x denotes the input of the first hidden layer (206) and the first hidden layer (206) is configured for dividing the outputs $<w_i.x>$ by $d=<|W_i|.|x|>$; and
an output layer (218) comprising a node associated with a numerical score which represents the predicted survival potential of the cell.

9. The machine-learning model of claim 7 or 8, wherein the ordinal regression model (120) comprises a loss function

$$ordinal_{loss(f,y)} = \max_{\forall(i,j)|y_i<y_j} (0,1 + f_i - f_j).$$

10. The use of an output dataset (122) produced by a machine-learning model according to any one of claims 6-9 for at least one of:

- predicting an effectiveness of a candidate agent for a degenerative disease;
- assessing the level of cytotoxicity in a population of cells;
- indicating the severity of a degenerative disease to improve prognosis;
- identifying which particular cell types are responding to a given treatment;
- identifying which particular cell types are affected by a disease;
- assessing the health status of a single cell.

11. A computer-implemented method (102) of training a machine-learning model configured for predicting the survival potential of a cell at single-cell resolution, in particular the machine-learning model of any one of claims 6-9, comprising:

receiving a training dataset (104), wherein the training dataset (104) comprises omics data associated with a plurality of cells and information on the health status of the plurality of cells;
training (108) the machine-learning model using data associated with the training dataset (104);
wherein the training dataset (104) is obtained in the context of a biological experiment of one of the following designs comprising:

- a perturbation experiment where beneficial and/or aggressive treatments are applied to cells, such as mechanical aggression and their transcriptomic response is followed over time together with their health status, such as e.g. cell death and/or cell proliferation;
- a regeneration experiment where transcriptomic profiles of cells from regenerative tissues are followed over time so the regenerative ability of a cell can be estimated from its transcriptomic profile;
- a degeneration experiment where the transcriptomic profiles of cells into degenerative diseases is compared or followed over time;
- an aging experiment where transcriptomic profiles of cells into organisms of increasing age is followed or compared between specimens so as to link the impact of age on the cell transcriptome;
- a developmental experiment where transcriptomic profiles of cells are followed during development of an organism.

12. The method of claim 11, further comprising:
generating (106) a gene expression matrix based on at least part of the training dataset (102), wherein the gene expression matrix encodes a normalized expression of a given gene in a given cell.

13. The method of claim 11 or 12, wherein the training (108) of the machine-learning model comprises:

subjecting a deep-learning model (118) of the machine-learning model to a first training phase, wherein the first training phase comprises a first plurality of epochs, preferably at least 30 epochs;

determining a pruning mask for a first hidden layer (206) of the deep-learning model (118), wherein the pruning mask is configured for selecting a first set of nodes with the highest weights and a second set of nodes with the lowest weights;

subjecting the deep-learning model (118) to a second training phase with the pruning mask applied, wherein the second training phase comprises a second plurality of epochs, preferably at least 30 epochs;

wherein, preferably, a dropout probability of the first hidden layer (206) involved in the first and/or second training step is selected from the range of 30% to 70%, more preferably from the range of 40% to 60%, and most probably is a dropout probability of essentially 50%.

14. A computer program comprising instructions which, when the program is executed by a computer, cause the computer to carry out the method of any one of claims 1-5 and/or 11-13.

15. A data processing system comprising means for carrying out the method of any one of claims 1-5 and/or 11-13.

Fig. 1

Input layer: Gene expression profile
one input per gene
202

118

Dropout layer, rate 50%
204

Custom linear layer: 1024 nodes
activation: hardtanh()
206

Normalization
adjustment
220

Dropout layer, rate 30%
208

Linear layer: 512 nodes
activation: relu()
210

Dropout layer, rate 30%
212

Linear layer: 256 nodes
activation: relu()
214

Dropout layer, rate 30%
216

Output layer: 1 node
Linear layer
218

Fig. 2

Fig. 3

log(Fold Change) of survival fraction after 15 days

Fig. 4

Fig. 5

**EUROPEAN SEARCH REPORT**

Application Number

EP 22 15 5787

## DOCUMENTS CONSIDERED TO BE RELEVANT

| Category | Citation of document with indication, where appropriate, of relevant passages | Relevant to claim |
|---|---|---|
| X | WO 2021/141374 A1 (KOREA ADVANCED INST SCI & TECH [KR]; PENTAMEDIX CO LTD [KR]) 15 July 2021 (2021-07-15) * 108, 116,117, fig. 1, claim 20 * | 1,5,6, 14,15 |
| X | WU ZHENYU ET AL: "Single-Cell Techniques and Deep Learning in Predicting Drug Response", TRENDS IN PHARMACOLOGICAL SCIENCES, ELSEVIER, HAYWARTH, GB, vol. 41, no. 12, 2 November 2020 (2020-11-02), pages 1050-1065, XP086341460, ISSN: 0165-6147, DOI: 10.1016/J.TIPS.2020.10.004 [retrieved on 2020-11-02] * p. 1053,1056,1054, fig. 1, I, table 1 * | 1,4, 7-11,13 |
| X | AARONM SMITH ET AL: "Standard machine learning approaches outperform deep representation learning on phenotype prediction from transcriptomics data", BMC BIOINFORMATICS, BIOMED CENTRAL LTD, LONDON, UK, vol. 21, no. 1, 20 March 2020 (2020-03-20) , pages 1-18, XP021274476, DOI: 10.1186/S12859-020-3427-8 * abstract, fig. 1 * | 1-3,12 |

-/--

**CLASSIFICATION OF THE APPLICATION (IPC)**

INV.
G16B25/10
G16B40/20

**TECHNICAL FIELDS SEARCHED (IPC)**

G16B

The present search report has been drawn up for all claims

| Place of search | Date of completion of the search | Examiner |
|---|---|---|
| Munich | 19 July 2022 | Lüdemann, Susanna |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

| | Europäisches Patentamt European Patent Office Office européen des brevets | **EUROPEAN SEARCH REPORT** | Application Number EP 22 15 5787 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PIRHAJI LEILA ET AL: "Identifying therapeutic targets by combining transcriptional data with ordinal clinical measurements", NATURE COMMUNICATIONS, vol. 8, no. 1, 1 December 2017 (2017-12-01), XP055943614, DOI: 10.1038/s41467-017-00353-6 Retrieved from the Internet: URL:https://www.nature.com/articles/s41467-017-00353-6.pdf> * p. 9, fig. 1 * | 1-15 | |

TECHNICAL FIELDS SEARCHED (IPC)

The present search report has been drawn up for all claims

| Place of search | Date of completion of the search | Examiner |
|---|---|---|
| Munich | 19 July 2022 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 4 227 948 A1

## ANNEX TO THE EUROPEAN SEARCH REPORT
## ON EUROPEAN PATENT APPLICATION NO.

EP 22 15 5787

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2022

| Patent document cited in search report | Publication date | Patent family member(s) | Publication date |
|---|---|---|---|
| WO 2021141374 A1 | 15-07-2021 | KR 20210089094 A | 15-07-2021 |
| | | WO 2021141374 A1 | 15-07-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021127610 A1
- WO 2021138548 A1
- US 20140057258 A1
- KR 102144719 B1
- CN 110222745 B
- WO 2021163706 A1

### Non-patent literature cited in the description

- **BROWN ; CAROLYN A. ; CATHY LALLY ; VARANT KUPELIAN ; W. DANA FLANDERS.** Estimated Prevalence and Incidence of Amyotrophic Lateral Sclerosis and SOD1 and C9orf72 Genetic Variants. *Neuroepidemiology,* 2021, vol. 55 (5), 342-53
- **WITTCHEN ; H.U. ; F. JACOBI ; J. REHM ; A. GUSTAVSSON ; M. SVENSSON ; B. JÖNSSON ; J. OLESEN et al.** The Size and Burden of Mental Disorders and Other Disorders of the Brain in Europe 2010. *European Neuropsychopharmacology,* 2011, vol. 21 (9), 655-79
- 2021 Alzheimer's Disease Facts and Figures. Alzheimer's Association, 2021, vol. 17
- **F. ARAI et al.** Machine Learning of Hematopoietic Stem Cell Divisions from Paired Daughter Cell Expression Profiles Reveals Effects of Aging on Self-Renewal. *Cell Syst.,* 08 December 2020, vol. 11 (6), 640-652
- **SZALAI ; BENCE ; VIGNESHWARI SUBRAMANIAN ; CHRISTIAN H HOLLAND ; ROBERT ALFÖLDI ; LÁSZLÓ G PUSKAS ; JULIO SAEZ-RODRIGUEZ.** Signatures of Cell Death and Proliferation in Perturbation Transcriptomics Data-from Confounding Factor to Effective Prediction. *Nucleic Acids Research,* 2019, vol. 47 (19), 10010-26
- **TEO et al.** Bundle Methods for Regularized Risk Minimization. JMLR, 2010
- **DO ; ARTIERES.** Regularized bundle methods for convex and non-convex risks. JMLR, 201